# EUROPEAN PATENT SPECIFICATION

(11) **EP 4 403 135 B1**
(45) Date of publication and mention of the grant of the patent: **26.11.2025**
(21) Application number: 24181074.6
(22) Date of filing: 19.10.2020
(51) Int. Cl.: A61F 2/95, A61F 2/07, A61F 2/962, A61F 2/06

(54) **SYSTEM FOR DEPLOYING AN IMPLANT**
SYSTEM ZUM EINSETZEN EINES IMPLANTATS
SYSTÈME DE DÉPLOIEMENT D'UN IMPLANT

(43) Date of publication of application: 24.07.2024
(62) Divisional of application: 20801166.8
(73) Proprietor: Angiomed GmbH & Co. Medizintechnik KG, 76227 Karlsruhe (DE)
(72) Inventor: Giebmeyer, Carsten, 76227 Karlsruhe (DE); Mair, Jutta, 76227 Karlsruhe (DE); Dorn, Jürgen, 76227 Karlsruhe (DE)
(74) Representative: Hoffmann Eitle

(56) References cited:
- US-A1- 2013 103 130
- US-A1- 2013 231 736
- US-A1- 2015 282 881
- US-A1- 2015 359 997
- US-A1- 2018 177 621

## Description

### Technical Field

The present invention relates to a system for deploying an implant, optionally a TIPS stent graft.

### Technical Background

In order to deploy implants such as stents and stent grafts inside a human body, it is generally known to use catheters that can be advanced through a patient's vasculature. At the distal end of the catheters, the implant to be delivered is arranged. This implant is typically crimped down on the delivery catheter and is covered with an implant retaining sheath that retains it in place. Once the implant has been positioned at the correct place inside the patient's body, the implant retaining sheath is retracted so that the implant can be deployed. In a number of cases, the implant is self-expanding, for example due to the use of a scaffolding made of a shape memory alloy such as nitinol, and will expand at body temperature. Accordingly, once such an implant is exposed to a patient's bloodstream, it will automatically expand to its delivery configuration.

US 2013/103130 A1 discloses a catheter with a handle mechanism having a pull force and tactile feedback for delivery of a stent-graft.

One particular type of implant that presents its own challenges are stent grafts for use in a TIPS ("transjugular intrahepatic portosystemic shunt") procedure. In a TIPS procedure, a shunt is created between the hepatic vein and the portal vein of a patient, with the shunt extending through the patient's liver. This shunt, which serves as a blood bypass between those two veins, is used to treat portal hypertension. To stabilize the shunt, a specialised TIPS stent graft is placed inside the shunt so as to extend from the portal vein to the hepatic vein.

A TIPS stent graft is unusual in that it is only partially covered: the covering material only covers one of the longitudinal ends whilst the respective other longitudinal end is uncovered and thus reveals the bare stent. This bare stent portion is then placed inside the portal vein and will typically expand (flare out) at that position. This flared part of the stent graft serves to anchor the TIPS stent graft inside the portal vein and thus keep it in place. Since, however, that part of the base stent is not covered, it will provide little obstruction to blood flow so that it will not stop blood from flowing into the stent graft from the portal vein.

Subsequently, the covered portion of the TIPS stent graft is deployed inside the shunt that has been created through the liver parenchyma so that the respective other end of the TIPS stent graft ends up in the hepatic vein. Accordingly, through the thus placed TIPS shunt, blood can bypass the liver and flow directly from the portal vein to the hepatic vein. This bypass alleviates portal hypertension.

One challenge with placing such stent grafts is that there is a need to accurately position the uncovered portion and the covered portion of the stent graft. On the one hand, it is important to ensure that the uncovered part of the stent graft is fully provided within the portal vein, since otherwise, blood could leak through the sidewalls of the TIPS stent graft. On the other hand, if parts of the covered section of the TIPS stent graft extend into the portal vein, they would at least partially obstruct blood flow and also reduce the effective length of the TIPS shunt that can be covered using the lined part of the TIPS stent graft. Accordingly, it is desirable to be able to accurately place the TIPS stent graft so that the uncovered portion is fully positioned within the portal vein, but without the covered portion of the TIPS stent graft extending into that vein.

### Summary of the Invention

The present invention has been conceived in view of the previously mentioned challenges and aims at providing a system for deploying an implant, optionally a TIPS stent graft, that makes it easier for a surgeon to correctly deploy that implant.

The invention is defined by claim 1. Embodiments are defined in the dependent claims.

According to claim 1, a system is provided for deploying an implant. Such an implant can, in embodiments according to the invention, be a TIPS stent graft. It can also be some other kind of stent or stent graft (covered stent).

The system comprises a handle that is that part of the system that is intended for manipulation by an operator. The system furthermore comprises a catheter that extends from the handle. The catheter is arranged for being introduced into a patient's vasculature so that it is sufficiently thin and flexible for being advanced through human blood vessels. At the distal end of the catheter, an implant holding portion is provided that is suitable for having the implant arranged thereon so that the implant can be held at the implant holding portion whilst advancing the catheter through a patient's vasculature to the implant deployment position.

Further provided is an implant retaining sheath that is arranged so as to surround the implant holding portion. When it surrounds the implant holding portion it will retain an implant that is arranged at the implant holding portion, for example by covering it. Such an implant retaining sheath can, in embodiments, be made of a suitable plastic material. The skilled person will be able to envisage other material that can be used.

Connected to the implant retaining sheath is a pull tab that could, in embodiments, be a pull string. This pull tab is arranged so that a proximal pull on the pull tab pulls back the implant retaining sheath so as to at least partially release an implant arranged at the implant holding portion. In the present context, "proximal" denotes a direction towards the operator of the system whilst "distal" denotes a direction away from the operator and towards the patient. The pull tab, which can, in embodiments, be made of a metal wire but could also be made of a string made of a non-metallic material or some other suitable element arranged for exerting a proximal tensile force on the implant retaining sheath, is therefore used for releasing the implant by gradually pulling back the implant retaining sheath.

The pull tab comprises an engagement feature that is arranged on that pull tab and that is moved proximally together with the pull tab when the pull tab is moved proximally. This engagement feature is arranged to engage with a first interference feature. This first interference feature is arranged at a first fixed longitudinal position on the system for deploying the implant. In embodiments, the engagement feature is provided at a fixed longitudinal direction on the handle.

The system is arranged so that an engagement between the engagement feature and the first interference feature provides tactile feedback to a user of the system that the implant retaining sheath has been pulled back a pre-set distance. That is, the user can feel, when pulling back the pull tab, that the implant retaining sheath has moved a certain pre-set distance. This aids in the partial deployment of an implant such as a TIPS stent graft since the user then, by the tactile feedback, knows that a pre-set distance of the implant retaining sheath has been pulled back and, accordingly, a pre-set portion of the implant has been uncovered and, in the context of a self-expanding implant, has expanded. Accordingly, the tactile feedback serves as an indication to the user that a pre-set portion of the implant has been uncovered and has thus been partially deployed . Whilst so far described mainly in terms of the deployment of a TIPS stent graft, the disclosure is also applicable to the deployment of bifurcated stent grafts for use in treating abdominal aortic aneurysms.

In embodiments, the system comprises a pull tab withdrawal mechanism which is a mechanism that is arranged on the system for aiding in pulling back the pull tab. In embodiments, the pull tab withdrawal mechanism is arranged on the handle. When the pull tab is pulled proximally using the pull tab withdrawal mechanism, an implant arranged at the implant holding portion is first partially and then completely uncovered. Such a pull tab withdrawal mechanism makes it easier for the clinician/operator to withdraw the implant retaining sheath.

In further embodiments, the pull tab withdrawal mechanism comprises a thumbwheel coupled to a spindle. The thumbwheel is operable for a user. The spindle is arranged for winding up the pull tab so as to exert a proximal pull on that pull tab. That is, by means of operating the thumbwheel, the pull tab is pulled proximally so as to pull the implant retaining sheath proximally to thereby release an implant arranged at the implant holding portion. In other embodiments, instead of using a thumbwheel, the pull tab is withdrawn by means of a slider that is used for pulling the pull tab back, such as described in WO 2008/017683 A1.

In embodiments, the tactile feedback provides a blocking of further withdrawal of the pull tab. This blocking of further withdrawal ensures that the point where the implant retaining sheath has been pulled back a pre-set distance cannot be missed by an operator since it would be impossible to withdraw the pull tab further. Accordingly, a corresponding system is highly user-friendly.

In further embodiments, the system further comprises a first release mechanism for selectively releasing the blocking of further withdrawal of the pull tab by the engagement between the engagement feature and the first interference feature. That is, the further withdrawal of the pull tab can be unblocked. Therefore, if the engagement between the engagement feature and the first interference feature corresponds to a partial deployment of the implant, the release of the blocking of further withdrawal of the pull tab can be advantageous in that one can then, subsequent to the partial removal of the implant retaining sheath, have a full removal of the implant retaining sheath. This aids in deploying an implant for which it is helpful that it is deployed in two stages, such as a TIPS stent graft.

In embodiments, the engagement feature comprises a protrusion that it is fixedly arranged on the pull tab. The first interference feature is a narrow opening provided inside the handle, where the opening is sized so that the protrusion engages with the boundaries of the opening to provide the tactile feedback to the user. Such a configuration of the engagement feature and the first interference feature has a high degree of reliability.

In embodiments, the pull tab is led through the opening provided inside the handle as part of the first interference feature. Such a configuration provides a good control of the path taken by the pull tab.

In embodiments, there is further provided a second interference feature that is arranged at a second fixed longitudinal position on the system for deploying an implant, where the second fixed longitudinal position is different from the first fixed longitudinal position. The engagement feature is arranged so as to engage with the second interference feature so as to provide tactile feedback to the user. Again, for reasons analogous to those set out regarding the first interference feature, this tactile feedback can provide useful information to the user, for example that the deployment of the implants would begin if the pull tab is retracted further (so that the engagement between the second interference feature and the engagement feature corresponds to a configuration where the implant retaining sheath covers the implant completely but where any further withdrawal of the implant retaining sheath would lead to the implant being partially uncovered).

In embodiments, the tactile feedback of the engagement between the engagement feature and the second interference feature is a blocking of further withdrawal of the pull tab which leads to the advantages described previously regarding the blocking of further withdrawal of the pull tab due to an engagement between the engagement feature and the first interference feature.

There is furthermore, in embodiments, provided a second release mechanism for selectively releasing the blocking of withdrawal of the pull tab by the engagement between the second interference feature and the engagement feature. This allows for selectively pulling the pull tab further back. The second release mechanism is optionally operable by means of a user operable button which makes for an easy-to-use system. Again, similar advantages to those discussed with respect to the first release mechanism can be achieved.

It is according to embodiments that the engagement between the second interference feature and the engagement feature corresponds to the implant retaining sheath fully covering the implant holding portion. That is, this engagement could be made to correspond to the initial configuration of the system (i.e. the configuration of the system it is meant to be in before being advanced through a patient's blood vessels).

According to embodiments of the invention, the system comprises a TIPS stent graft, that is arranged at the implant holding portion. With such a system, an implant such as a TIPS stent graft can be suitably delivered.

In embodiments, the implant is a self-expanding implant. By this, it is meant that the implant will expand to a fully expanded configuration when warmed up to the body temperature experienced inside a human or animal body. Such a self-expanding implant can comprise a nitinol scaffolding as an example of a shape memory alloy. However, other types of material can also be used.

In embodiments, the engagement between the engagement feature and the first interference feature corresponds to a partial deployment of the implant. This can make it easy to suitably place the implant inside a patient's body. In embodiments where a TIPS stent graft is provided on the implant holding portion, the engagement between the engagement feature and the first interference feature would correspond to the uncovered portion of the TIPS stent graft being deployed whilst the cover portion is still covered by the implant retaining sheath.

### Brief Description of the Drawings

Figure 1 shows a system according to an embodiment of the invention.
Figure 2 shows an interior of the handle of the system of Figure 1 in a first configuration.
Figure 3 shows the interior of Figure 2 and a second configuration.
Figure 4 shows an interior of the system of Figure 1 in a third configuration.
Figure 5 shows the steps that can be taken during deployment of a TIPS stent graft.

### Detailed Description of the Drawings

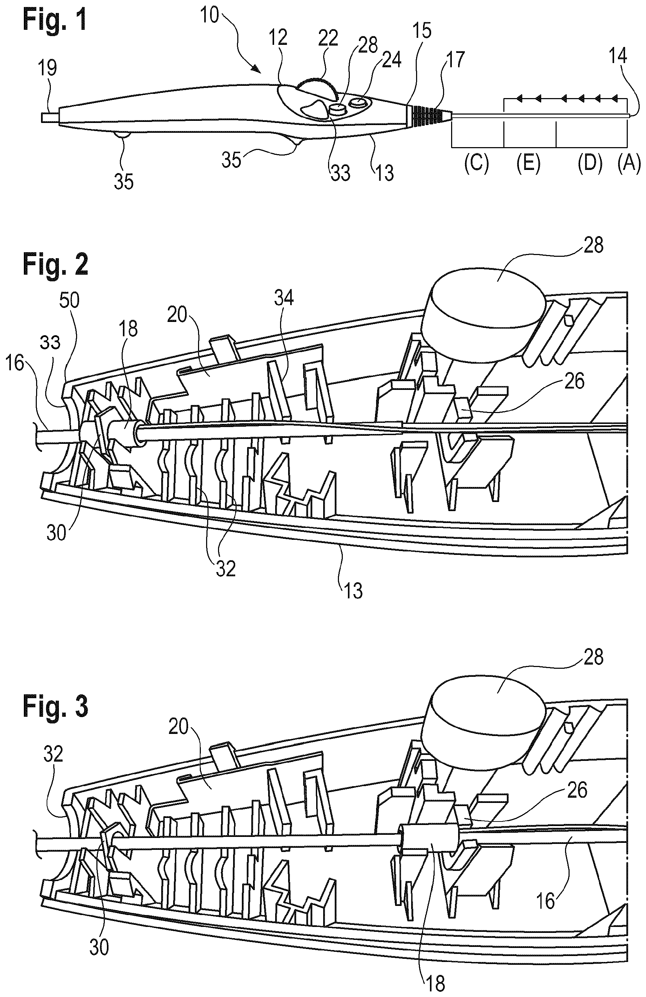

Figure 1 shows a system for deploying an implant according to an embodiment of the invention. As can be seen from this Figure, a handle 12 is provided that comprises a first component 13 and a second component 15 that are arranged on top of each other and that are connected to one another. At one end of the handle 12, a flexible tip 17 is provided. At the end of the flexible tip 17, a catheter 14 that is suitable for insertion into a patient's vasculature is arranged. This catheter 14 can be advanced through a patient's vasculature and can provide, at the distal end thereof, a TIPS stent graft. The catheter 14 has provided thereon an implant holding portion (not shown) at the distal end thereof as well as an implant retaining sheath (not shown) that covers the implant holding portion.

The handle 12 has provided on its upper surface a recess 33 that is arranged so that a surgeon can rest his or her thumb thereon. At the opposite side of the handle 12 relative to the recess 33, two protrusions 35 are provided that are arranged so as to help the surgeon in holding the device and the handle and to prevent it from slipping. A surgeon can grip with his or her hand around handle 12 so that his or her thumb rests on the recess 33 whilst the remaining fingers are arranged between the protrusions 35 so that the handle 12 is securely held. The protrusions 35 are provided at two longitudinal spaced apart positions of the handle 12.

A thumbwheel 22 is arranged on the handle 12 so that it sticks out of an opening of the handle 12 that is provided inside the recess 33. The thumbwheel 22 has a structured surface that increases friction thereof so as to make operating it easier. The thumbwheel 22 is coupled to a spindle (not shown) that is, in turn, coupled to the pull tab 16 that will be discussed further below with reference to Figures 2-4. This pull tab 16 is coupled to the implant retaining sheath so that a proximal movement of the pull tab 16 will pull the implant retaining sheath proximally to thus uncover the implant holding portion and to thus deploy an implant that is held at the implant holding portion. By operating the thumbwheel 22, the pull tab 16 will be wound onto the spindle so that the pull tab 16 is pulled proximally (to the left hand side in Figure 1).

In Figure 1, a first release mechanism 28 and a second release mechanism 24 are provided. The first release mechanism 28 is to be actuated by means of a button whilst the second release mechanism is operable by means of a slider. When the second release mechanism 24 has not been operated, a structure to be discussed below with reference to Figure 2-4 will block a withdrawal of the implant retaining sheath so that the implant holding portion cannot even be partially uncovered. That is, unless the second release mechanism 24 has been actuated, an implant provided at the implant portion cannot even be partially deployed. However, when sliding the slider of the second release mechanism 24 to the actuated position, this blocking of proximal withdrawal of the pull tab 16 will be released so that thereby, a rotation of the thumbwheel 22 can pull the pull tab 16 proximally. This allows for a partial deployment of an implant that is arranged at the implant holding portion at the distal end of the catheter 14.

When the pull tab 16 is pulled further proximally by means of operating the thumbwheel 22, the proximal pulling of the pull tab 16 will be blocked by an engagement with a first interference feature 26 to be discussed further below. Also that engagement can be selectively released by means of actuating the first release mechanism 28 by means of pushing the associated button. This releasable blocking of further withdrawal of the pull tab 16 can correspond to a partial deployment of the implant arranged at the distal end of the catheter 14. In the case of a TIPS stent graft provided at the distal end of the catheter 14, this can correspond to the selective and partial release of the uncovered portion of that stent graft. As pointed out before, this is highly advantageous when it comes to deploying and placing a TIPS stent graft in a human liver. Once the uncovered portion has been deployed, the surgeon can actuate the first release mechanism to release the blocking of further withdrawal of the pull tab 16 to thus completely release the implant.

In the structure shown in Figure 1, different positions and portions have been marked up on the catheter 14. The position A denotes the position up to which the implant retaining sheath extends when the implant holding portion is fully covered. This position corresponds to the position where the further withdrawal of the pull tab 16 is blocked by means of the second release mechanism 24 not being actuated. When the second release mechanism 24 is actuated, the implant retaining sheath can be pulled back over a distance denoted by the letter "D". This partial withdrawal corresponds to only the uncovered portion of, e.g., a TIPS stent graft being exposed to the patient's body.

The portion denoted by E corresponds to a sufficiently full withdrawal of the implant retaining sheath that the entirety of the implant holding portion (and thus of the implant) is uncovered so that the implant can be deployed. This would correspond to the full deployment configuration.

The portion denoted by C is that portion of the catheter 14 that is not used for carrying the implant. That is, this portion serves to provide enough of a length of the catheter 14 so as to reach the deployment position of the implant.

The catheter 14 can have provided therein a lumen suitable for leading a guidewire therethrough. This guidewire can extend, at the proximal end of the handle 12, through a connector 19 that can be a Luer connector.

Figure 2 shows an inside view of the lower part 13 of the handle 12 in a configuration where the implant retaining sheath extends up to the point A shown in Figure 1. That is, the implant holding portion is fully covered. The lower part 13 of the handle 12 is made of a plastic shell that has a semi-tubular shape that bulges out when going from the distal end 50 towards the central portion. This allows for ergonomically gripping the handle 12. At the distal end 50, a hemispherical cut out 32 is provided through which the pull tab 16 extends. This pull tab 16 is led through the interior of the lower part 13 and is to be rolled up on a spindle (not shown) attached to thumbwheel 22.

Provided inside the lower part 13 are reinforcing ribs 32 that serves so as to provide mechanical stability to the lower part 13. Those reinforcing walls 32 have a notch through which the pull tab 16 is led. That is, those notches serve to stabilise the path of the pull tab 16. Close to the distal end 50 of the lower part 13 is provided a metallic plate 30 having a hole provided therein through which the pull tab 16 is led. Also this plate 16 serves to stabilise the path taken by the pull tab 16. This plate 30 is attached (clipped) to one of the ribs 32.

Provided proximally relative to the plate 30 is a leaf spring 20 that is, at one of its ends, held by ribs 34 provided on a wall of the lower part 13. This leaf spring 20 is bent. Its bent portion abuts against an engagement feature 18 in the form of a ring like protrusion fixedly arranged on the pull tab 16. The bent portion of the leaf spring 20 abuts against the proximal-most portion of the engagement feature 18 so that it prevents a proximal movement of the engagement feature 18. In turn, this prevents a proximal movement of the pull tab 16. The leaf spring 20 is coupled to the slider 24 so that a sliding movement of the slider 24 will bend the leaf spring 20 so that the bent portion gets out of engagement with the engagement feature 18. In that configuration, a proximal movement of the engagement feature 18 is possible so that the pull tab 16 can be moved proximally. This then allows for a pulling back of the implant retaining sheath across the portion denoted by the region "D" in Figure 1. The bent portion of the leaf spring 20 thus constitutes the second interference feature.

Provided even further proximally relative to the leaf spring 20 is the first interference feature 26 that has the shape of a U-shaped plastic plate that is coupled to a pushbutton 28. The pull tab 16 is led through the recess of the U-shaped plate 26. When the pushbutton 28 is depressed, the U-shaped plate 26 is pushed down sufficiently far that the pull tab 16 no longer extends through the recess of the U-shaped plate 26. The recess of the U-shaped plate 26 is sized so that the engagement feature 18 cannot fit through it so that the engagement between the engagement feature 18 and the U-shaped plate 26 blocks a movement of the engagement feature 18 through that recess whilst being sufficiently wide to allow the rest of the pull tab 16 to freely pass through it.

Figure 3 shows a configuration where the engagement between the second interference feature in the form of the leaf spring 20 and the engagement feature 18 had been released and where the pull tab 16 has been pulled proximally so that the implant retaining sheath has moved across the portion D in Figure 1. As can be seen from Figure 3, in that configuration, the engagement feature 18 now abuts against the first interference feature 26. Since the pushbutton 28 has not been depressed, a proximal movement of the engagement feature 18 is prevented by that abutment.

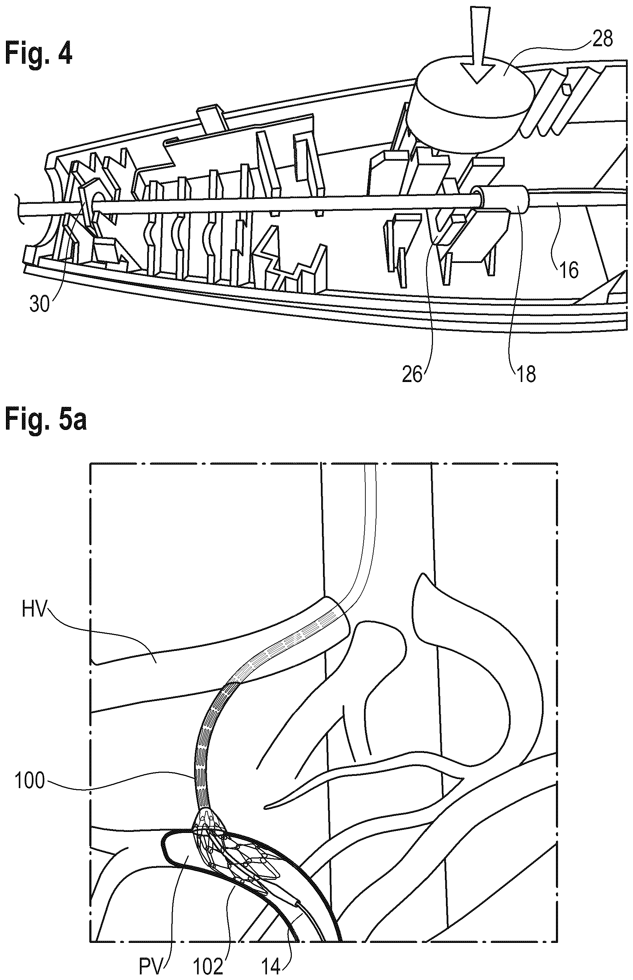

In the configuration that is shown in Figure 4, the pushbutton 28 has been depressed, as indicated by an arrow, so that there is now no longer an engagement between the first interference feature 26 and the engagement feature 18. Accordingly, it is possible to pull the pull tab 16 proximally so that also the engagement feature 18 can now be pulsed to a position that is proximal to now arranged proximally of the first interference feature 26. In that configuration, the implant retaining sheath can be moved across the region E shown in Figure 1 to fully deploy the implant that is provided on the implant holding portion.

Thus, with the device described previously, a surgeon is given clear tactile feedback as to when implant retaining sheath withdrawal begins and when the implant has been partially uncovered. The surgeon could thus, once he or she knows that the implant has been partially uncovered, ensure that the thus uncovered portion is properly placed.

For example in the case of a TIPS stent graft, the surgeon could ensure that the flared uncovered portion of the TIPS stent graft is arranged in the portal vein, for example using X-ray imaging of markers that are provided on the TIPS stent graft. The surgeon could then feel that the flared portion is completely arranged inside the portal vein by noticing the flared portion abutting against the junction between the TIPS shunt and the portal vein (as a mechanical resistance against proximal withdrawal of the TIPS stent graft to be deployed). Accordingly, since the surgeon knows that only the uncovered portion has been uncovered but that this portion has been uncovered completely, a highly accurate and user-friendly positioning of the TIPS stent graft is possible.

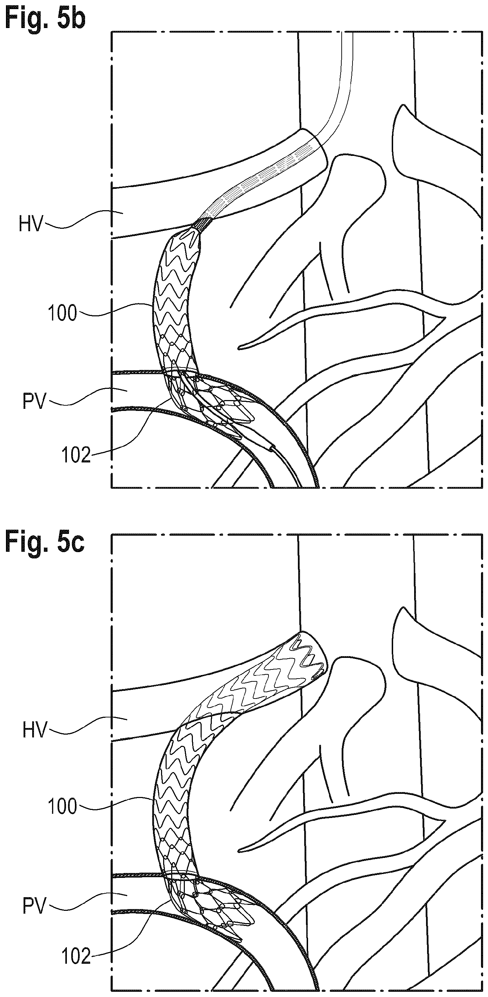

Accordingly, the following steps would be taken by a surgeon when wishing to place a TIPS stent graft using the previously described design, which are also partially illustrated in Figures 5a)-c):
- Creating a TIPS shunt connecting a patient's hepatic (HV) and portal (PV) veins,
- Inserting the catheter 14 into the patient's vasculature and advance its tip - in embodiments using a guidewire (not shown) - via the TIPS shunt to the portal vein,
- Releasing the blocking of withdrawal of the pull tab 16 by the engagement between the second interference feature 20 and the engagement feature 18 (not shown),
- Withdrawing the implant retaining sheath so as to release the uncovered portion 102 of the TIPS stent graft 100 until further withdrawal is blocked by the engagement between the first interference feature 26 and the engagement feature 18 (Figure 5a)),
- Positioning the uncovered portion of the TIPS stent graft inside the portal vein PV,
- Releasing the blocking of further withdrawal of the pull tab 16 by the engagement between the first interference feature 27 and the engagement feature 18,
- Withdrawing the implant retaining sheath so as to fully release the TIPS stent graft 100 (Figures 5b), c)).

It is to be noted that whilst the present invention has been mainly described in terms of the placement of a TIPS stent graft, it is also equally applicable to the placement of bifurcated stent grafts for the treatment of abdominal aortic aneurysms.

## Claims

1. System (10) for deploying an implant, the system comprising:
- a handle (12),
- a catheter (14) extending from the handle (12), the catheter being arranged for holding an implant at an implant holding portion at a distal end of the catheter,
- an implant retaining sheath, the implant retaining sheath being arranged so as to surround the implant holding portion so as to retain an implant that is arranged at the implant holding portion,
- a pull tab (16), the pull tab (16) being connected to the implant retaining sheath so that a proximal pull on the pull tab pulls back the implant retaining sheath so as to release an implant arranged at the implant holding portion,
the pull tab (16) further comprising an engagement feature (18) that is arranged on the pull tab (16) and that is moved proximally as the pull tab (16) is moved proximally, the engagement feature (18) being arranged to engage with a first interference feature (26) arranged at a first fixed longitudinal position on the system for deploying the implant, wherein an engagement between the engagement feature (18) and the first interference feature (26) provides tactile feedback to a user of the system that the implant retaining sheath has been pulled back a pre-set distance,
the system further comprising a TIPS stent graft arranged at the implant holding portion.

2. System according to claim 1, further comprising a pull tab withdrawal mechanism, the pull tab withdrawal mechanism being arranged pull the pull tab (16) proximally so as to release an implant arranged at the implant holding portion.

3. System according to claim 2, the pull tab withdrawal mechanism comprising a thumbwheel (22) coupled to a spindle, the spindle being arranged for winding up the pull tab (16) so as to exert a proximal pull on the pull tab (16).

4. System according to one of the preceding claims, wherein the tactile feedback is a blocking of further withdrawal of the pull tab (16).

5. System according to claim 4, the system further comprising a first release mechanism (28) for selectively releasing the blocking of further withdrawal of the pull tab (16), the first release mechanism (28) optionally being operable by a user operable button.

6. System according to one of the preceding claims, wherein the engagement feature (18) comprises a protrusion that is fixedly provided on the pull tab (16) and wherein the first interference feature (26) comprises an opening provided inside the handle (12), with the opening being sized so that the protrusion engages with the boundaries of the opening to provide tactile feedback to the user.

7. System according to one of the preceding claims, further comprising a second interference feature (20) that is arranged at a second fixed longitudinal position on the system for deploying the implant, the engagement feature (18) being arranged to engage with the second interference feature (20) so as to provide tactile feedback to the user, wherein the tactile feedback is optionally a blocking of withdrawal of the pull tab (16).

8. System according to claim 7, the system further comprising a second release mechanism (24) for selectively releasing the blocking of withdrawal of the pull tab (16) by the engagement between the second interference feature (20) and the engagement feature (18), the second release mechanism (24) optionally being operable by means of a slider.

9. System according to claim 7 or 8, the engagement between the second interference feature (20) and the engagement feature (18) corresponding to the implant retaining sheath fully covering the implant holding portion.

10. System according to one of the preceding claims, the implant being a self-expanding implant.

11. System according to one of the preceding claims, wherein the engagement between the engagement feature (18) and the first interference feature (26) corresponds to a partial deployment of the implant.

## Patentansprüche

1. System (10) zum Einsetzen eines Implantats, wobei das System Folgendes umfasst:
- einen Griff (12),
- einen Katheter (14), der sich vom Griff (12) erstreckt, wobei der Katheter zum Halten eines Implantats an einem Implantathalteabschnitt an einem distalen Ende des Katheters angeordnet ist,
- eine Implantatrückhaltehülle, wobei die Implantatrückhaltehülle dazu angeordnet ist, den Implantathalteabschnitt zu umgeben, um ein Implantat, das am Implantathalteabschnitt angeordnet ist, zurückzuhalten,
- eine Zuglasche (16), wobei die Zuglasche (16) mit der Implantatrückhaltehülle verbunden ist, sodass ein proximaler Zug an der Zuglasche die Implantatrückhaltehülle zurückzieht, um ein Implantat freizugeben, das am Implantathalteabschnitt angeordnet ist,
wobei die Zuglasche (16) weiter ein Eingreifmerkmal (18) umfasst, das auf der Zuglasche (16) angeordnet ist und das proximal bewegt wird, wenn die Zuglasche (16) proximal bewegt wird, wobei das Eingreifmerkmal (18) zum Eingreifen in ein erstes Interferenzmerkmal (26) angeordnet ist, das an einer ersten festen Längsposition an dem System zum Einsetzen des Implantats angeordnet ist, wobei ein Eingreifen zwischen dem Eingreifmerkmal (18) und dem ersten Interferenzmerkmal (26) eine taktile Rückmeldung an einen Benutzer des Systems bereitstellt, dass die Implantatrückhaltehülle um einen voreingestellten Abstand zurückgezogen wurde,
wobei das System weiter einen TIPS-Stent-Graft umfasst, der am Implantathalteabschnitt angeordnet ist.

2. System nach Anspruch 1, weiter umfassend einen Zuglaschenrückziehmechanismus, wobei der Zuglaschenrückziehmechanismus dazu angeordnet ist, die Zuglasche (16) proximal zu ziehen, um ein am Implantathalteabschnitt angeordnetes Implantat freizugeben.

3. System nach Anspruch 2, wobei der Zuglaschenrückziehmechanismus ein Daumenrad (22) umfasst, das mit einer Spindel gekoppelt ist, wobei die Spindel zum Aufziehen der Zuglasche (16) dazu angeordnet ist, einen proximalen Zug auf die Zuglasche (16) auszuüben.

4. System nach einem der vorstehenden Ansprüche, wobei die taktile Rückmeldung ein Blockieren von weiterem Zurückziehen der Zuglasche (16) ist.

5. System nach Anspruch 4, wobei das System weiter einen ersten Freigabemechanismus (28) zum selektiven Freigeben des Blockierens von weiterem Zurückziehen der Zuglasche (16) umfasst, wobei der erste Freigabemechanismus (28) optional durch einen benutzerbetätigbaren Druckknopf betätigt werden kann.

6. System nach einem der vorstehenden Ansprüche, wobei das Eingreifmerkmal (18) einen Vorsprung umfasst, der fest auf der Zuglasche (16) bereitgestellt ist, und wobei das erste Interferenzmerkmal (26) eine Öffnung umfasst, die innerhalb des Griffs (12) bereitgestellt ist, wobei die Öffnung so bemessen ist, dass der Vorsprung in die Grenzen der Öffnung eingreift, um taktile Rückmeldung an den Benutzer bereitzustellen.

7. System nach einem der vorstehenden Ansprüche, weiter umfassend ein zweites Interferenzmerkmal (20), das an einer zweiten festen Längsposition am System zum Einsetzen des Implantats angeordnet ist, wobei das Eingreifmerkmal (18) zum Eingreifen in das zweite Interferenzmerkmal (20) dazu angeordnet ist, taktile Rückmeldung an den Benutzer bereitzustellen, wobei die taktile Rückmeldung optional ein Blockieren von Zurückziehen der Zuglasche (16) ist.

8. System nach Anspruch 7, wobei das System weiter einen zweiten Freigabemechanismus (24) zum selektiven Freigeben des Blockierens von Zurückziehen der Zuglasche (16) durch das Eingreifen zwischen dem zweiten Interferenzmerkmal (20) und dem Eingreifmerkmal (18) umfasst, wobei der zweite Freigabemechanismus (24) optional mittels eines Schiebers betätigt werden kann.

9. System nach Anspruch 7 oder 8, wobei das Eingreifen zwischen dem zweiten Interferenzmerkmal (20) und dem Eingreifmerkmal (18) einem vollständigen Abdecken der Implantatrückhaltehülle durch den Implantathalteabschnitt entspricht.

10. System nach einem der vorstehenden Ansprüche, wobei das Implantat ein selbstexpandierendes Implantat ist.

11. System nach einem der vorstehenden Ansprüche, wobei das Eingreifen zwischen dem Eingreifmerkmal (18) und dem ersten Interferenzmerkmal (26) einem partiellen Einsetzen des Implantats entspricht.

## Revendications

1. Système (10) de déploiement d'un implant, le système comprenant :
- une poignée (12),
- un cathéter (14) s'étendant depuis la poignée (12), le cathéter étant agencé pour maintenir un implant au niveau d'une partie de maintien d'implant à une extrémité distale du cathéter,
- une gaine de retenue d'implant, la gaine de retenue d'implant étant agencée de manière à entourer la partie de maintien d'implant de manière à retenir un implant qui est agencé au niveau de la partie de maintien d'implant,
- une languette de traction (16), la languette de traction (16) étant connectée à la gaine de retenue d'implant de sorte qu'une traction proximale sur la languette de traction tire en arrière la gaine de retenue d'implant de manière à libérer un implant agencé au niveau de la partie de maintien d'implant,
la languette de traction (16) comprenant en outre un élément de mise en prise (18) qui est agencé sur la languette de traction (16) et qui est déplacé de manière proximale lorsque la languette de traction (16) est déplacée de manière proximale, l'élément de mise en prise (18) étant agencé pour venir en prise avec un premier élément d'interférence (26) agencé à une première position longitudinale fixe sur le système de déploiement de l'implant, dans lequel une mise en prise entre l'élément de mise en prise (18) et le premier élément d'interférence (26) fournit un retour tactile à un utilisateur du système indiquant que la gaine de retenue d'implant a été tirée en arrière sur une distance prédéfinie,
le système comprenant en outre un stent-greffe TIPS agencé au niveau de la partie de maintien d'implant.

2. Système selon la revendication 1, comprenant en outre un mécanisme de retrait de languette de traction, le mécanisme de retrait de languette de traction étant agencé pour tirer la languette de traction (16) de manière proximale de manière à libérer un implant agencé au niveau de la partie de maintien d'implant.

3. Système selon la revendication 2, le mécanisme de retrait de languette de traction comprenant une molette (22) couplée à une broche, la broche étant agencée pour enrouler la languette de traction (16) de manière à exercer une traction proximale sur la languette de traction (16).

4. Système selon l'une des revendications précédentes, dans lequel le retour tactile est un blocage d'un retrait supplémentaire de la languette de traction (16).

5. Système selon la revendication 4, le système comprenant en outre un premier mécanisme de libération (28) pour libérer sélectivement le blocage d'un retrait supplémentaire de la languette de traction (16), le premier mécanisme de libération (28) étant facultativement actionnable par un bouton actionnable par l'utilisateur.

6. Système selon l'une des revendications précédentes, dans lequel l'élément de mise en prise (18) comprend une saillie qui est prévue de manière fixe sur la languette de traction (16) et dans lequel le premier élément d'interférence (26) comprend une ouverture prévue à l'intérieur de la poignée (12), avec l'ouverture qui est dimensionnée de sorte que la saillie vienne en prise avec les limites de l'ouverture pour fournir un retour tactile à l'utilisateur.

7. Système selon l'une des revendications précédentes, comprenant en outre un deuxième élément d'interférence (20) qui est agencé au niveau d'une deuxième position longitudinale fixe sur le système de déploiement de l'implant, l'élément de mise en prise (18) étant agencé pour venir en prise avec le deuxième élément d'interférence (20) de manière à fournir un retour tactile à l'utilisateur, dans lequel le retour tactile est facultativement un blocage d'un retrait de la languette de traction (16).

8. Système selon la revendication 7, le système comprenant en outre un deuxième mécanisme de libération (24) pour libérer sélectivement le blocage d'un retrait de la languette de traction (16) par l'a mise en prise entre le deuxième élément d'interférence (20) et l'élément de mise en prise (18), le deuxième mécanisme de libération (24) étant facultativement actionnable au moyen d'un curseur.

9. Système selon la revendication 7 ou la revendication 8, la mise en prise entre le deuxième élément d'interférence (20) et l'élément de mise en prise (18) correspondant à la gaine de retenue d'implant recouvrant entièrement la partie de maintien d'implant.

10. Système selon l'une des revendications précédentes, l'implant étant un implant auto-expansible.

11. Système selon l'une des revendications précédentes, dans lequel la mise en prise entre l'élément de mise en prise (18) et le premier élément d'interférence (26) correspond à un déploiement partiel de l'implant.
